# EUROPEAN PATENT APPLICATION

(11) **EP 2 457 648 A1**
(43) Date of publication of application: **30.05.2012**
(21) Application number: 10192974.3
(22) Date of filing: 29.11.2010
(51) Int. Cl.: B01J 8/04, C11C 3/00

(54) **Production of fatty acid alkyl esters**

(71) Applicant: Yellow Diesel B.V., 1018 WB Amsterdam (NL)
(72) Inventor: Dimian, Alexandre C., 1183 EC Amstelveen (NL); Rothenberg, Gadi, 2341 NV Oegstgeest (NL); Schut, Ronald, 1183 LP Amsterdam (NL)
(74) Representative: De Vries & Metman

(57) **Abstract**

Process and reactor for the production of fatty acid alkyl esters, wherein a feedstock is fed to a reactor system comprising at least one set (24) of a plurality of tubular reactors (21, 22, 23). Each tubular reactor has a flow-through channel provided with one or more heterogeneous catalysts. The tubular reactors are in a serial arrangement The reactor comprises valves (27, 33) for selectively connecting or disconnecting at least one of the tubular reactors to or from the serial arrangement.

## Description

The present invention relates to a process and a reactor system for the production of fatty acid alkyl esters, in particular for use as biodiesel.

Biodiesel is essentially a mixture of esters of fatty acids with alkyl alcohols, mostly as fatty acid methyl esters (FAME). They are obtained by the esterification of fatty acids with alcohols and/or by the transesterification of triglycerides present in the lipid raw materials, as vegetable oils and fats, with the same alcohols.

Homogeneous catalysis - acid for esterification and base for transesterification - and batch processes dominate the manufacturing processes. The key advantage is that the operation conditions in a batch reactor, most of all the reaction time, can be easily adapted to the specific kinetics of various raw materials in order to achieve the strict quality specifications of biodiesel, as defined by EN 14142. Beside inherently low productivity, a major drawback of a batch process is the generation of waste streams due to the removal of catalyst, either by the production of salt by neutralization and water washing, or by adsorption/desorption on porous solid materials. The cleaning of biodiesel from additional chemicals is costly and energy intensive. Another major drawback is that the glycerol grade, obtained as a by-product, is hardly useful since containing water and salts. To put it in a nutshell, the separation and purification steps by batch processes using homogeneous catalyst are rather complex and cost ineffective.

For the above reasons using heterogeneous catalysis seems very attractive having the potential of substantial savings at least in the hardware, by suppressing the need of vessels for the above mentioned steps, but also in energy consumption, mainly by suppressing costly waste water evaporation and recycling. Moreover, since glycerol of high purity is obtained as valuable by-product, the overall profitability is substantially improved.

Despite these significant advantages, only one major implementation of heterogeneous catalysis for large scale manufacturing of bio-diesel is known, which is the Esterfip-H process by Axens. The explanation might be in the fact that getting long-run robust and active catalyst is difficult. The Esterfip-H process makes use of a catalyst on the basis of zinc aluminate, as described in US 7,151,187. The Esterfip-H process makes use of tall tower reactors designed for higher pressure and temperatures. Since the variation in the residence time of such reactors is limited by hydrodynamics and mass transfer requirements, it comes out the design should be tailored to the use of a certain feedstock quality only. In addition, replacement or regeneration of deactivated catalyst should imply large amount of materials, special tools and costly manpower.

In biodiesel manufacturing by heterogeneous catalysis chemistry and process design are interrelated. The residence time needed to achieve a given degree of conversion depends on the composition of the feedstock in term of saturated and non-saturated fatty acid pattern.

In general, heterogeneously catalyzed transesterification can take place in a tubular reactor wherein a feedstock of oil and alcohol is provided to the inlet of a reactor comprising a solid catalyst. The reactor content flows from the inlet side in a continuous flow towards the outlet where it leaves the reactor as an effluent comprising fatty acid alkyl esters, glycerol, and, depending on the degree of conversion, smaller or larger amounts of fatty acid glycerol esters and residual alcohol.

The required reaction time, and thus the required residence time in the reactor, varies with the type and composition of the feedstock. Hitherto, the residence time in tubular reactors of a given length, such as column-type reactors or serpentine-type reactors, can be varied by varying the flow speed. However, the flow speed can be varied only within a limited range in view of hydrodynamic and mass transfer preconditions. Consequently, a specific tubular reactor can only be used for a limited number of feedstock types.

The tubular reactors contain a heterogeneous catalyst to catalyze the transesterification reaction. After a certain period of use the catalyst needs to be replaced and/or regenerated. Conventionally, replacement of catalyst in tubular reactors, such as single fixed bed or tubes-and-shell reactors, requires shut-down of the complete reactor, while the catalyst should be discharged and replaced as a whole, causing substantial economic loss.

It is an object of the invention to provide a reactor system for the production of a biodiesel which is suitable for a larger range of feedstock types. Preferably, shut-down time of the reactor for replacement of catalyst should substantially be reduced, and preferably be prevented.

The object of the invention is achieved with a process for the production of fatty acid alkyl esters, wherein a feedstock is fed to a reactor system comprising at least one set of a plurality of tubular reactors, each tubular reactor having a flow-through channel comprising solid catalyst, the tubular reactors being in a serial arrangement, the reactor system comprising one or more valves for selectively connecting or disconnecting at least one of the tubular reactors to or from the serial arrangement.

In this way, the residence time within the reactor system as a whole can easily be adapted by adapting the number of active tubular reactors in the serial arrangement. The flow velocity of the reactor content can be kept constant at an optimal value that maximizes the catalyst efficiency. Optimal hydraulic conditions, mass transfer rate and catalyst integrity can be preserved. If a specific feedstock requires a shorter residence time, one or more of the tubular reactors can be switched off and be by-passed. If a longer residence time is desired, the number of active tubular reactors can be increased. Since a larger range of residence times can be achieved, the reactor system as a whole can be used for a wide range of feedstock types.

Moreover, if the catalyst in one of the reactors need to be replaced, the reactor in question can easily be isolated and detached while the process can still be continued in the other tubular reactors. Complete shutdown of the reactor system as a whole is not required anymore.

The reactor system enables continuous operation for biodiesel production by heterogeneous catalysis. The reactor can for example be built as a single box or container provided with piping connections for reactants, products and utility fluids and can for example be plugged in into an existing biodiesel production facility. The box can be isolated to minimize heat loss.

In a particular embodiment, a reactor system is used comprising a plurality of tubular reactors each having an inlet and an outlet wherein the outlet of at least one of the tubular reactors is connected to a first line with a first branch leading to the inlet of a second tubular reactor and a second branch leading to the inlet of a third tubular reactor, the line comprising a valve for selectively closing off and opening the first or second branch. A second line connects the outlet of the second reactor to the inlet of the third tubular reactor.

In one embodiment, the tubular reactors can be arranged side by side forming a meandering, serpentine process flow path. This may allow close positioning of the reactors together, which saves space and makes for easy implementation.

In one embodiment the tubular reactors can be arranged next to each other, e.g., horizontally or vertically. This allows a compact positioning of the reactors and easy loading and unloading of catalyst.

The same or different process conditions can apply within the various tubular reactors. For instance, the temperature and/or the pressure can increase or decrease stepwise per tubular reactor, if so desired.

Each tubular reactor can be provided with control means for controlling one or more process parameters, such as mean residence time, pressure and/or temperature. Alternatively, the reactor system as a whole may have a central control unit, controlling these process parameters centrally for a number of tubular reactors or for all tubular reactors. This option has the advantage of cost saving.

During operation the temperature within a tubular reactor will generally be in the range of 100°C to 300°C, e.g., 150°C to 250°C, e.g., 180°C to 220°C. The pressure within the tubular reactors will generally be within the range of 5 to 60 bar, e.g., 15 to 50 bar, e.g. 25 to 40 bar. The liquid hourly space velocity (LHSV) within the tubular reactor will generally be the same for all reactors connected in series, but may be made to differ if so desired by adjusting the reactor diameter. The LHSV is generally in the range of 0.2 to 5, in particular 0.5 to 2 m³/h input charge / m³ catalyst.

The feedstock comprises one or more oils and/or animal fats and one or more alcohols. These oils typically are triglycerides of C8 - C22 fatty acids. Suitable oils include rapeseed oil, soybean oil, sunflower oil, palm oil, coconut oil, jatropha oil, karanja oil, cameline oil, algae oil, or waste vegetable oil (WVO). Suitable animal fats include tallow, lard, yellow grease, chicken fat, and the by-products of the production of omega-3 fatty acids from fish oil. In one embodiment the feedstock contains at least 50 wt.% of triglycerides of C10-C18 fatty acids, in particular at least 70%, more in particular at least 80%, calculated on the weight amount of oils and/or animal fats. Suitable alcohols include methanol, ethanol, propanol, butanol, iso-propanol or mixtures thereof. The use of methanol is considered preferred.

The molar ratio between alcohol and triglyceride as it is provided to the reactor system is generally at least stoichiometric, e.g. between 3:1 and 7:1, while the maximum is typically dependent on reaction conditions and the type of catalyst used. An excess of alcohol (e.g., when the molar alcohol: triglyceride ratio is at least 3.05:1) is preferably employed, to improve the reaction rate and to compensate for alcohol lost in separation steps. Such an alcohol excess can for instance be 10 - 30 vol.% for batch processes or 40 - 60 vol.% for heterogeneous processes. However, the use a large excess of alcohol (e.g., when the molar alcohol: triglyceride ration is at least 5:1) is generally not useful and not of interest for economic reasons, and often not of interest for economic reasons. It is in fact one of the advantages of the present invention that a relatively low excess of alcohol can be used, due to higher possible flow speeds and better mass transfer conditions. Accordingly, the molar ratio between alcohol and triglyceride is preferably in the range of 3.05 to 5, more specifically 3.1 to 4.

The tubular reactors contain one or more catalysts suitable for catalyzing the transesterification process. The solid catalyst can for instance be in the form of grains, extrudates or pellets of any form or size. The solid catalysts can, e.g., be basic or acidic or be a mixture of basic and acidic catalysts. Particular examples for acid catalysts are montmorillonite clays, heteropolyacids, sulphated and/or phosphated oxides, suich as zirconia, titania, vanadia or niobia or mixed oxides. Particular examples of base catalysts are hydrotalcites, layered double hydroxides and solids comprised of basic functionalities, such as amino groups supported on carbon or on inorganic supports, such as silica, alumina and/or zirconia. Optionally, the catalysts can be mixed with one or more inert materials. Optionally, the different tubular reactors can contain different catalysts of different composition, particle size, porosity or activity.

The number of tubular reactors in a serial arrangement can for instance be 2, 3 or 4 or more. The optimum number of reactors depends, among others, on the catalyst activity, the desired conversion rate, the selectivity and the length and diameter of the tubular reactors. As a maximum number of tubular reactors connected in series a general value 20 may be mentioned. It should be noted that the reactor system can comprise additional tubular reactors which are not connected in series during the operation, to allow switching of the reactors, e.g. for catalyst regeneration or replacement.

The tubular reactors in the serial arrangement can all have the same length and/or have the same diameter. Alternatively, they can all have the same length with different diameters or have the same diameter with different lengths, or have different diameters and different lengths. To allow easy replacement of the individual tubular reactors, it may be preferred for all reactors to have the same dimensions, that is a length which is within 10% of the mean length of all tubular reactors present, and a diameter which is within 10% of the mean diameter of all tubular reactors present.

Generally, the length of a tubular reactor within the serial arrangement is within the range of 1 m to 10 meter. A length in the range of 1.5 to 3 meter is presently considered preferred because it combines ease of use with process efficiency. However, the tubular reactors can also be larger or smaller if so desired.

Generally, the diameter of a tubular reactor within the serial arrangement is within the range of 5 cm to 50 cm. A range of 10 cm to 20 cm is presently considered preferred for flow control and heat exchange. However, the tubular reactors can also have larger or smaller diameters, if so desired.

Generally, the length to diameter ratio L/D of a tubular reactor within the serial arrangement is within the range of 10:1 to 100:1. However, larger or smaller ratios can also be used, if so desired.

In a particular embodiment, the reactor system may comprise a feed line with branches for each one of the tubular reactors, the branches leading to the inlet of a respective tubular reactor, the branches being provided with a three-way valve for selectively closing off the inlet or downstream sections of the feed line, wherein the reactor system further comprises a discharge line connected to the outlet of each tubular reactor, wherein between at least one pair of adjacent tubular reactors the feed line is connected to the discharge line by a return line, wherein the discharge line is provided with valves for selectively closing the return line or downstream sections of the discharge line.

The process of transesterification can be promoted by the pre-heating the feedstock before it enters the tubular reactors. This heat can be recovered after the mixture leaves the serially arranged tubular reactors. Energy can be saved by heat recovery with a feed effluent heat exchanger (FEHE) upstream the tubular reactors. The feed effluent heat exchanger comprises a tubular conduit jacketed with a cylindrical hollow sleeve forming a downstream section of the discharge line. In the heat exchanger, fresh feedstock is heated by heat from freshly processed reactor content, which is consequently cooled down, e.g., for further processing steps, e.g., to a temperature within the range of 40°C to 70°C. The inlet of the sleeve is arranged near the outlet of the tubular reactor outlet and the outlet of the sleeve is arranged near the inlet of the tubular reactor inlet, in order to increase heat exchange by a counter current arrangement. The use of such a feed effluent heat exchanger can save 70 - 85 % of the required total amount of energy required for preheating the mixture.

If so desired, supplementary heating and/or cooling devices can be integrated in the reactor system for controlling the reaction and/or separation steps, for instance heat exchangers using a fluid heating medium or liquid thermal agent (LTA). In one embodiment, the system comprises both a FEHE and a supplementary heating device serially arranged.

The heat exchangers used in the reactor system are preferably compact and can for example be made of soldered or screwed plate. The heat exchangers should preferably be capable of handling fluids with a maximum pressure of, e.g., 30 - 50 bars and temperatures up to 250 - 300 °C. A multi-pass compact heat exchanger can be designed to accommodate both feed effluent heat exchange and LTA heat exchange in the same device, if so desired.

Optionally, the feed line can provided with one or more homogenizers, such as a static mixer. The homogenizer(s) can be positioned between two adjacent tubular reactors or upstream the tubular reactors, for instance upstream the heat exchanger and/or between the heat exchanger and the tubular reactors.

The conversion rate of the transesterification process can be increased by drawing off glycerol during the process. To this end, the reactor system may for example comprise two or more sets of tubular reactors and a glycerol separator having an inlet connected to the end of the discharge line of a first set of tubular reactors, a first outlet connected to a feed line of a second set of tubular reactors, and a second outlet for the discharge of separated glycerol. This way, the equilibrium of the transesterification can be shifted to obtain a conversion rate higher than 90 %, e.g., higher than 99 %.

The glycerol separator can for example be a coalescence separator, e.g., making use of gravity. A bed of hydrophilic material, such as glass fibers, alumina, zeolites, etc. and/or hydrophobic materials, such as polymer fibers, can be used to accelerate the coalescence process and the formation of larger drops.

Separation is facilitated by the large difference in density of the ester and glycerol phases and by the immiscibility of fatty acid alkyl esters and glycerol. The non-reacted alcohol is distributed between the ester and glycerol phases with preference for the last one. If there is no substantial excess of alcohol the heavy phase contains glycerol and alcohol with only traces of esters, while the light phase contains esters with only a small amount of glycerol not substantially affecting a next transesterification step. The initial weight ratio alcohol:oil should be such that after the separation of the glycerol sufficient alcohol remains to complete the second stage transesterification. To this end, the initial alcohol content can for instance be at least 20 wt.%, e.g., in the range of 20 - 35 wt.% by weight of the total feedstock.

Alternatively, or additionally, membrane filtration can be used to remove undesired components in the reactor content. For instance, the reactor content can first be separated by membrane filtration into a first residue comprising at least the largest part of the fatty acid glycerol ester content, and an intermediate filtrate which is subsequently separated in a second membrane filtration step using an alcohol selective filter membrane into a filtrate comprising at least the largest part of the alcohol content, and a second residue. This second residue mainly comprises fatty acid alkyl esters, glycerol and residual alcohol. The residual alcohol can be removed by distillation. After further removal of glycerol, a crude grade biodiesel remains. This way, an integrated reaction-separation apparatus can be obtained for the manufacturing of biodiesel by heterogeneous solid catalysis.

With the features described above combined the reactor system enables continuous operation for biodiesel production by heterogeneous catalysis as an integrated system of reaction, energy saving and in-situ separation of products and/or reactants.

The present invention will be elucidated with reference to the figures, wherein:
- Figure 1:: schematically shows an embodiment of a reactor for carrying out a process according to the invention in a flow diagram;
- Figure 2:: schematically shows an alternative embodiment of a reactor for carrying out a process according to the invention in a flow diagram;
- Figure 3:: shows a further alternative embodiment of a reactor for carrying out a process according to the invention in a flow diagram.

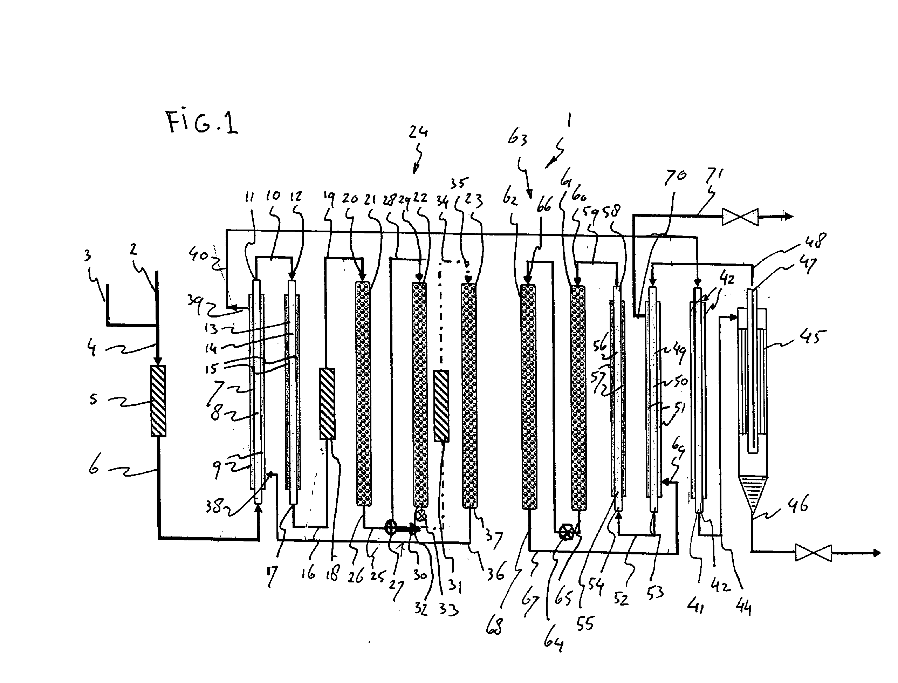

Figure 1 shows a flow diagram representing an embodiment of a reactor system 1 according to the present invention. The reactor system 1 is used for the production of fatty acid alkyl esters for use in biodiesel by transesterification of a vegetable oil or animal fat using an alcohol, such as methanol.

The reactor system 1 comprises an oil inlet 2 and an alcohol inlet 3 joining to form a first feed line section 4 to a static mixer 5. A second feed line section 6 connects the static mixer 5 to a first heat exchanger 7. The first heat exchanger 7 comprises a central channel 8 connected to the second feed line section 6, and a jacket 9 packing the channel 8 in a heat conductive manner. A third feed line section 10 connects the outlet 11 of the channel 8 to the inlet 12 of a second heat exchanger 13. The second heat exchanger 13 comprises a central channel 14 connected to the second feed line section 6, and a jacket 15 packing the central channel 14 in a heat conductive manner. The jacket 15 of the second heat exchanger is connected to a source of a heating fluid (not shown) or liquid thermal agent (LTA).

A fourth feed line section 16 connects the outlet 17 of the second heat exchanger 13 to a second static mixer 18. A fifth feed line section 19 leads from the static mixer 18 to the inlet 20 of a first tubular reactor 21 of a first set 24 of tubular reactors 21, 22, 23. The three tubular reactors 21, 22, 23 define a channel provided with a heterogeneous catalyst for promoting the transesterification process. A sixth feed line section 25 runs from the outlet 26 of the tubular reactor 21 to a three-way valve 27, where the feed line branches into a seventh feed line section 28 running to the inlet 29 of the second tubular reactor 22, and an eighth feed line section 30 running to a third static mixer 31. The second tubular section 22 comprises an outlet 32 with a close-off valve 33. The outlet 32 is connected to the eighth feed line section 30. A ninth feed line section 34 runs from the third static mixer 31 to the inlet 35 of the third tubular reactor 23. A tenth feed line section 36 runs from the outlet 37 of the third tubular reactor 23 to the inlet 38 of the jacket 9 of the first heat exchanger 7. The jacket 9 comprises an outlet 39 connected to an eleventh feed line section 40 which leads to a utility cooler 41 with a water cooling 42, although air cooling can also be used. The fin cooler 41 comprises an outlet 43 connected to a twelfth feed line section 44, forming a discharge line for the first set 24 of tubular reactors and leading to a coalescence separator 45 for the separation of glycerol. The coalescence separator 45 comprises a first outlet 46 for glycerol discharge and a second outlet 47 connected to a thirteenth feed line section 48. The thirteenth second feed line section 48 leads to a third heat exchanger 49 comprising a central channel 50 connected to the thirteenth feed line section 48, and a jacket 51 packing the central channel 50 in a heat conductive manner. A fourteenth feed line section 52 connects the outlet 53 of the channel 50 to the inlet 54 of a fourth heat exchanger 55. The fourth heat exchanger 55 comprises a central channel 56 connected to the fourteenth feed line section 52, and a jacket 57 packing the central channel 56 in a heat conductive manner. The jacket 57 of the fourth heat exchanger 55 is connected to a source of a heating fluid (not shown). The central channel 56 has a terminal outlet 58 connected to a fifteenth feed line section 59 leading to the inlet 60 of a first tubular reactor 61 of a second set 63 of tubular reactors 61, 62. The two tubular reactors 61, 62 define a channel provided with a heterogeneous catalyst for promoting the transesterification process. A sixteenth feed line section 64 runs from the outlet 65 of the tubular reactor 61 to the inlet 66 of the second tubular reactor 62 of the second set 63. A seventeenth feed line section 67 runs from the outlet 68 of the second tubular reactor 62 of the second set 63 to the inlet 69 of the jacket 51 of the third heat exchanger 49. The jacket 51 comprises an outlet 70 connected to a discharge line 71.

In the subsequent tubular reactors 21, 22, 23, 61, 62 the mixture is subjected to a transesterification process to form a mixture of unreacted alcohol, fatty acid alkyl esters, glycerol and fatty acid glycerol esters.

A feedstock is supplied to feed line 4 of the reactor system 1. The feedstock is transported to the first and second heat exchangers 7, 13 to heat the mixture to a temperature favourable for the transesterification process in the downstream tubular reactors 21, 22, 23. For example, in the first heat exchanger, the temperature may be raised from 20°C to 180°C, and in the second heat exchanger the temperature may be raised from 20°C to 250°C, to reach reaction conditions, e.g., a temperature within the range of 100°C to 300°C, e.g., 150°C to 250°C, e.g., 180°C to 220°C. The feedstock is then transported further to the first tubular reactor 21 of the first set 24 of tubular reactors, where the transesterification process is started. If the feedstock is of a type that requires a longer residence time, the three-way valve 27 is switched to open the seventh feed line section 28 running to the inlet 29 of the second tubular reactor 22, where the transesterification process is continued. Close-off valve 33 is also opened, so the mixture can continue flowing through the eighth feed line section 30, the third static mixer 31 and the ninth feed line section 34 to the inlet 35 of the third tubular reactor 23, where the transesterification process is continued again.

If the feedstock is of a type that requires a shorter residence time, the three-way valve 27 is switched to close off the seventh feed line section 28 and to open the eighth feed line section 30. This way, the second tubular reactor 22 is disconnected from the serial arrangement of the set 24 of tubular reactors and the close-off valve 33 is closed. The mixture flows directly through the eighth feed line section 30, the third static mixer 31 and the ninth feed line section 34 to the inlet 35 of the third tubular reactor 23, where the transesterification process is continued. This way, the residence time is only 2/3 of the residence time when the three-way valve is switched to connect the second tubular reactor 22 to the serial arrangement.

After having passed the third tubular reactor 23, the mixture is transported via the tenth feed line section 36 to the inlet 38 of the jacket 9 of the first heat exchanger 7. In the heat exchanger heat is transferred from the mixture in the jacket 9 to fresh feedstock in the central channel 8 of the first heat exchanger. When passing the jacket 9 the temperature of the mixture can for instance fall to a temperature within the range of 40°C to 70°C. Dependent on the applied residence time, catalyst activity and other process conditions, the conversion rate at this stage can generally be within the range of 70 to 90 wt.%. The mixture is cooled to enable more effective separation of glycerol in the downstream coalescence separator 45. Therefore, the mixture is first fed to the cooler 41, where heat is dissipated. Here the temperature of the mixture falls to a temperature within the range of, e.g., 30°C to 80°C °C, preferably 40°C to 60°C.

Subsequently, the mixture enters the coalescence separator 45 where a mixture of glycerol and alcohol is drawn off from the mixture and discharged via the glycerol outlet 46. Depending on the settling time and coalescence conditions, the glycerol content in the fatty acid alkyl ester mixture can be reduced by 70 - 90 wt.% by total weight of the mixture before separation, which is sufficient or shifting the chemical equilibrium to over 99 % in a next transesterification cycle.

The coalescence separator can for instance be designed for a superficial fluid velocity (with respect to transversal area) of 0.5 - 2 mm/s, which in the frame of the present invention can give settling times of 10 - 40 minutes.

The remaining mixture leaves the coalescence separator 45 via the outlet 47 and is then transported to the third heat exchanger 49 and subsequently to a fourth heat exchanger 55, where the mixture is heated again to a temperature favourable for the transesterification process, e.g., a temperature within the range of 100°C to 300°C, e.g., 150°C to 250°C, e.g., 180°C to 220°C. Subsequently, the mixture is fed to the tubular reactors 61 and 62 respectively, where the transesterification process is continued. Due to the reduced glycerol content in the mixture, a very high conversion rate can be achieved with the transesterification of mono-, di- and triglycerides and alcohol into glycerol and the desired fatty acid alkyl esters during this phase, e.g. of 99 % or more.

Subsequently, the mixture is fed to the jacket 57 of the fourth heat exchanger 55. The fourth heat exchanger 55 is a utility heater, preferably driven by a liquid thermal agent (LTA).

Finally, the mixture is discharged via the discharge line 71. The final triglyceride conversion rate can be more than 98 wt.%, e.g. more than 99,5 wt.%

After the reaction stage before separation of the glycerol, the content of fatty acid alkyl esters is generally within the range of 50 to 80 wt.%, the glycerol content of the mixture is generally within the range of 2 to 5 wt.%, the alcohol content is generally within the range of 5 to 45 wt.% and the oil content is generally within the range of 5 to 30 wt.% (all percentages by weight being calculated on the total weight of the mixture), dependent on the type of feedstock and other conditions. These components can be separated in one or more further process steps, for example by membrane filtration.

For instance, the mixture can first be separated by membrane filtration into a first residue or retentate comprising at least the largest part of the fatty acid glycerol ester content, and an intermediate filtrate or permeate which is subsequently submitted to a second membrane filtration step using an alcohol selective filter membrane into a filtrate comprising at least the largest part of the alcohol content, and a second residue or retentate. This second residue mainly comprises fatty acid alkyl esters, glycerol and residual alcohol. In this mixture a heavy phase with a high glycerol content can be separated more easily from a phase with a high fatty ester content by conventional techniques such as L-L decanting or centrifugation. The residual alcohol can be removed from one or both effluents by distillation to produce biodiesel and high purity glycerol.

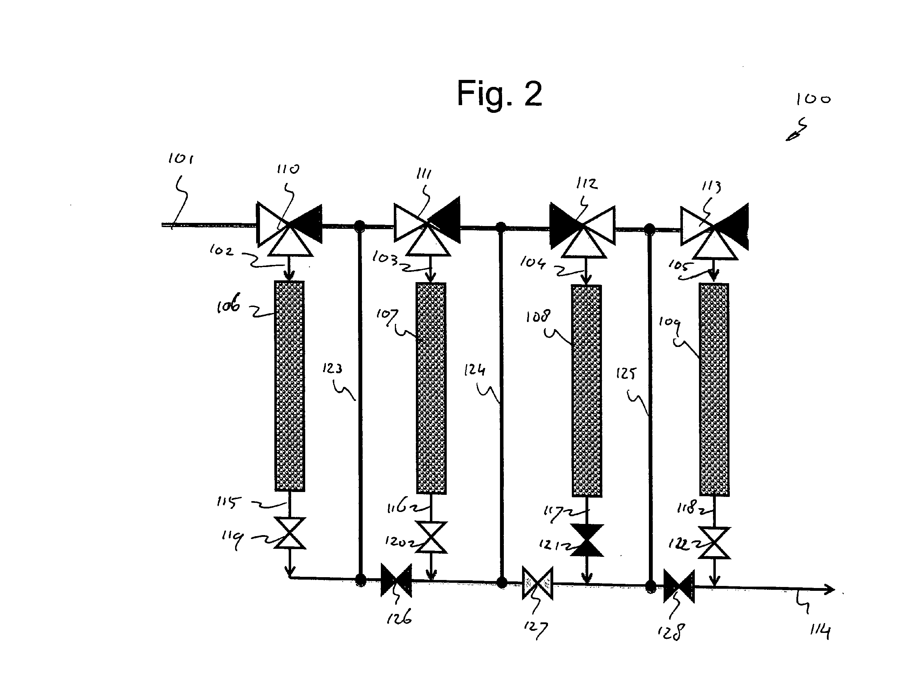

An alternative embodiment of a reactor 100 according to the invention is shown schematically in Figure 2. In this embodiment, the reactor system comprises a feed line 101 with branches 102, 103, 104, 105, each leading to a tubular reactor 106, 107, 108, 109. The four tubular reactors 106, 107, 108, 109 define a channel provided with a heterogeneous catalyst for promoting the transesterification process. Each one of the branches 102, 103, 104, 105 is provided with a three way valve 110, 111, 112, 113 for selectively closing off the respective tubular reactor 106, 107, 108, 109 or downstream sections of the feed line 101. The reactor system 100 further comprises a discharge line 114 connected to the outlet 115, 116, 117, 118 of each tubular reactor 106, 107, 108, 109. Each outlet 115, 116, 117, 118 is provided with a close-off valve 119, 120, 121, 122. Between each pair of adjacent tubular reactors the feed line 101 is connected to the discharge line 114 by a return line 123, 124, 125. At short distance downstream each return line 123, 124, 125, the discharge line 114 is provided with a valve 126, 127, 128 for selectively closing or opening downstream sections of the discharge line.

If a short residence time is required, valve 110 is switched to open reactor 106. The other three-way valves 111, 112, 113 are closed, while the close-off valves 126, 127, 128 in the discharge line 114 are opened. This way, mixture passing the first tubular reactor 106 is directly discharged for further processing.

If a double residence time is required, valve 126 in the discharge line 114 is closed off, while the other two valves 127, 128 in the discharge line 114 remain open. Three-way valve 11 of the second tubular reactor 107 is switched to open the reactor 107 in serial arrangement with the first tubular reactor 106. Mixture passing the first reactor 106 is routed via return line 123 via three way valve 111 to the second tubular reactor 107. Subsequently, the mixture is discharged via the discharge line 114.

In a similar way, the third and the fourth tubular reactor 108, 109 can be connected to the serial arrangement for further increase of the residence time.

After a certain processing time, the catalyst within the tubular reactors 106, 107, 108, 109 need to be replaced. When the catalysts in a given tubular reactor needs to be replaced, the respective three-way valve 110, 111, 112, 113 at the inlet of the tubular reactor in question can be switched to close off the tubular reactor and to open the downstream section of the feed line 101. The corresponding valve 119, 120, 121, 122 at the outlet of the tubular reactor is also closed. Subsequently, the tubular reactor in question is detached from the arrangement. The catalyst content is removed and replaced by fresh catalyst. Subsequently, the tubular reactor is put back in place and the valves can be switched to reconnect the tubular reactor to the serial arrangement. This way, the tubular reactors can be refilled one by one. Meanwhile the process can be continued in the remaining tubular reactors, without the need to shut-down the complete reactor system.

By way of example the reactor system of Figure 2 contains only four tubular reactors. However, the number of tubular reactors can be less than four or more than four if so desired. In principle, the number of tubular reactors is not limited. In practice, the umber of tubular reactors is determined by the required total reactor volume, which is in turn determined by the reaction kinetics and the final conversion and selectivity. For example, a base module comprising two serpentines each with fifteen tubular reactors with a diameter of 150 mm and a length of 2.2 m filled with tested solid catalyst can yield a biodiesel production of about 7600 liter per day, or 2250 ton per year.

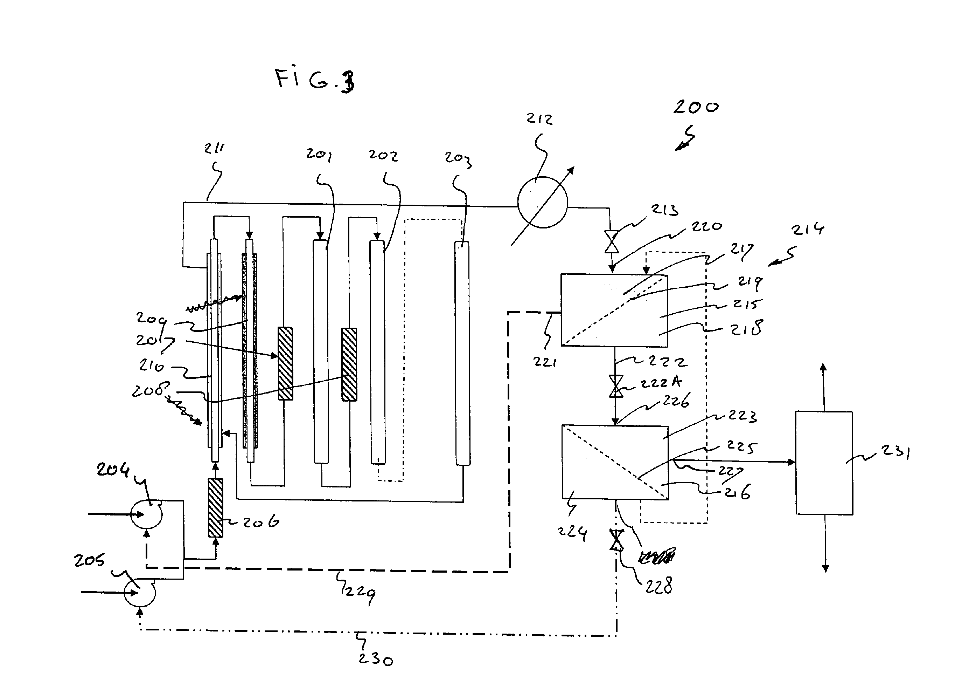

Figure 3 shows a further possible embodiment of a reactor system according to the invention. The reactor 200 comprises a set of serially arranged tubular reactors 201, 202, 203, oil and alcohol inlets 204, 205, static mixers 206, 207, 208, a pre-heater 209, and a feed effluent heat exchanger 210 in a similar arrangement as with the embodiment of Figure 1. A discharge line 211 transports cooled reactor content from the feed effluent heat exchanger 210 via a cooler 212 and a valve 213 for pressure reduction to a filter unit 214 for a two-step membrane filtration.

The kinetic diameter of methanol is 0.38-0.41 nm and the kinetic diameter of glycerol of 0.63 nm. The kinetic diameter of fatty acid alkyl esters depends on the length and unsaturated character of the hydrocarbon chain, and is generally in the range of 0.8 to 1.5 nm. The size of triglyceride molecules is almost by an order of magnitude larger. These size differences enable easy separation of unconverted glycerides molecules from fatty acid alkyl esters, glycerol and methanol. In addition, there are differences in hydrophobic / hydrophilic character that can be exploited for membrane separation.

The filtration unit 214 comprises a first filter 215 and a second filter 216. The first filter 215 comprises a retentate space 217, a permeate space 218 and a filter membrane 219 separating the retentate space 217 from the permeate space 218. The retentate space 217 has an inlet 220 which is operatively connected to the discharge line 211. The retentate space 217 also comprises a retentate outlet 221. The permeate space 218 comprises a permeate outlet 222 with a valve 222A. The filter membrane 219 is selective for fatty acid alkyl esters, alcohol and glycerol. The average pore size in the filter membrane should be sufficient to retain at least the major pat of the mono-, di- and triglycerides.

If the volumetric ratio methanol/triglyceride oil is larger than 40% then a micro-emulsion can be formed with oil as dispersed phase. The average micelle size is typically in the range 12 to 400 µm. Without micro-emulsion the pore size of the membrane should preferably be in the range 5 to 20 nm, in the case of micro-emulsion formation, the average pore size can preferably be in the range of 200 to 1000 nm.

The permeate outlet 222 of the permeate space 218 is operatively connected to the second filter 216. Conform the first filter 215, the second filter 216 comprises a second retentate space 223, a second permeate space 224 and an alcohol selective filter membrane 225 separating the second retentate space 223 from the second permeate space 224. The filter membrane 225 is alcohol selective. If the alcohol is methanol, filter membrane 22 has an average pore diameter of 0,5 - 5 nm, e.g., 0,7 - 1 nm. The second retentate space 223 comprises an inlet 226 and a second retentate outlet 227. The second permeate space 224 comprises a second permeate outlet with a valve 228.

The filter membranes 219, 225 can be hydrophilic or hydrophobic membranes, depending on the operating pressure, temperature and feedstock used. The membranes can for instance be made from polymeric materials, as polytetrafluoroethylene (PTFE), polypropylene, polyimide, polysulpholane, polycarbonate, etc., as well as from inorganic materials, such as porous ceramic, silica, α-alumina, microporous glass tubes, glass hollow fibres or mixtures thereof. Ceramic membranes offer higher chemical and thermal stability.

A first return line 229 connects the retentate outlet 221 of the first filter 215 to the oil inlet 204. Likewise, a second return line 230 connects the second permeate outlet 228 of the second filter 216 to the alcohol inlet 205.

Under the action of the pump 212 the reactor content is transported via the valve 213 into the retentate space 217. The mixture is separated into a retentate comprising the major part of the fatty acid mono-, di- and triglycerides content, and a permeate passing the membrane filter 219 to arrive in the permeate space 218. The permeate comprises a mixture of fatty acid alkyl esters, glycerol and alcohol.

The retentate comprising the major part of the fatty acid mono-, di- and triglycerides is passed via the retentate outlet 221 and the return line 229 to the oil inlet 204. The permeate comprising the mixture of fatty acid alkyl esters, glycerol and alcohol is transported to the second filter 219, where it enters the second retentate space 217. The second filter 219 can, e.g., be a nano-filtration filter or a pervaporation filter. If liquid-phase nano-filtration is used, the upstream and downstream pressures can be controlled by the bubble point of the inlet mixture and the permeate mixture. The temperature can be controlled by means of the cooler 212. If pervaporation is used the downstream pressure should be below the bubble point at the filtration temperature. A condenser (not shown) should be placed on the alcohol return line 230. Further energy saving can be achieved by heat exchange with the cold oil charge.

Most of the alcohol, e.g., at least 80 wt.% of the alcohol content of the permeate of the first filter 215, will pass the alcohol selective membrane 225 to arrive in the permeate space 224 as a permeate, which is recycled to the alcohol inlet 205 via the second return line 230. A mixture of the fatty acid alkyl esters, glycerol and residual alcohol will leave the retentate space 223 via the retentate outlet 227 to a further separation unit 231. In this stage the glycerol content of the mixture is generally within the range of 8 to 10 wt.% and the fatty acid alkyl ester content is generally within the range of 85 to 90 wt.% with some residual amounts of alcohol. In separation unit 231, the mixture is separated into a fatty acid alkyl ester stream and a glycerol stream, for instance by L-L decanting or centrifugation. Residual alcohol in the fatty ester and glycerol streams can be removed easily, e.g., by vapour/liquid equilibrium separations, such as flash evaporation, stripping or distillation in a short column. High quality biodiesel and high purity glycerol can be obtained without the need of further separation or purification steps.

Optionally, the reactor system 200 may comprise a return line 232 for recycling alcohol separated by the second filter 216 to further improve the effectivity of the first separation step.

## Claims

1. Process for the production of fatty acid alkyl esters, wherein a feedstock comprising triglyceride and alcohol is fed to a reactor system comprising at least one set (24) of a plurality of tubular reactors (21, 22, 23), each tubular reactor having a flow-through channel provided with one or more heterogeneous catalysts for catalyzing the process, the tubular reactors being in a serial arrangement, the reactor system having and one or more valves (27, 33) for selectively connecting or disconnecting at least one of the tubular reactors to or from the serial arrangement.

2. Process according to claim 1 wherein the tubular reactors (21, 22, 23) each have an inlet (20, 29, 35) and an outlet (26, 32, 37) wherein the outlet (20) of at least one of the tubular reactors is connected to a line (25) with a first branch (28) leading to the inlet (29) of a second tubular reactor (22) and a second branch (30) leading to the inlet (35) of a third tubular reactor (23), the line (25) comprising a valve (27) for selectively closing off and opening the first or second branch, and wherein a second line (34) connects the outlet (32) of the second reactor (22) to the inlet (35) of the third tubular reactor (23).

3. Process according to claim 1 or 2 wherein the reactor system (100) comprises a feed line (101) with branches (102, 103, 104, 105) for each one of the tubular reactors (106, 107, 108, 109), the branches leading to the inlet of a respective tubular reactor, the branches being provided with a valve (110, 11, 112, 113) for selectively closing off the inlet or downstream sections of the feed line, wherein the reactor system further comprises a discharge line (114) connected to the outlet of each tubular reactor, wherein between at least one pair of adjacent tubular reactors the feed line is connected to the discharge line by a return line (123, 124, 125), wherein the discharge line is provided with valves (126, 127, 128) for selectively closing off one or more of the return lines or downstream sections of the discharge line.

4. Process according to any one of the preceding claims wherein the reactor content flows along a heat exchanger (7, 49) upstream the tubular reactors, the heat exchanger comprising a tubular central channel (8, 50) jacketed in a heat conductive manner with a hollow sleeve (9, 51) forming a downstream section of the discharge line.

5. Process according to claim 4 wherein the hollow sleeve (9, 51) forms part of a discharge line for reactor content that has passed the tubular reactors connected to the serial arrangement.

6. Process according to claim 4 or 5 wherein the reactor content is homogenized by at least one homogenizer (5, 18, 31), such as a static mixer, upstream the heat exchanger (7) and/or between the heat exchanger (7) and the tubular reactors (21, 22, 23).

7. Process according to any one of the preceding claims wherein after the reactor content has passed the connected tubular reactors, it passes one or more separators for the separation of the reactor content into at least two different phases and wherein one of the phases is reused in a further transesterification step.

8. Process according to claim 7 wherein glycerol is separated from the reactor content and wherein the remaining reactor content is fed to a following set (63) of a plurality of tubular reactors for continued transesterification.

9. Process according to claim 8 wherein the glycerol is separated with a coalescence separator (45).

10. Process according to claim 8 or 9 wherein the reactor content is cooled before it is fed into the separator.

11. Reactor system for a process according to any one of the preceding claims comprising at least one set (24) of a plurality of tubular reactors (21, 22, 23) with a flow-through channel provided with one or more heterogeneous catalysts for the transesterification process, the tubular reactors being in a serial arrangement, wherein the reactor system comprises one or more valves (27, 33) for
selectively connecting or disconnecting at least one of the tubular reactors to or from the serial arrangement.

12. Reactor according to claim 11 wherein the tubular reactors (21, 22, 23) each have an inlet (20, 29, 35) and an outlet (26, 32, 37) wherein the outlet (20) of at least one of the tubular reactors is connected to a line (25) with a first branch (28) leading to the inlet (29) of a second tubular reactor (22) and a second branch (30) leading to the inlet (35) of a third tubular reactor (23), the line (25) comprising a valve (27) for selectively closing off and opening the first or second branch, and wherein a second line (34) connects the outlet (32) of the second reactor (22) to the inlet (35) of the third tubular reactor (23).

13. Reactor according to claim 11 or 12 wherein the reactor system comprises at least two sets (24, 63) of a plurality of tubular reactors and a glycerol separator (45) having an inlet (47) connected to the end of a discharge line (44) of the first set of tubular reactors (24), a first outlet for the discharge of separated glycerol and a second outlet (47) connected to a feed line of the second set (63) of tubular reactors.

14. Method of replacing a catalyst in a transesterification reactor according to any one of the preceding claims 11 - 13 wherein the valves are turned to a position to isolate the tubular reactor which is subsequently disconnected from the serial arrangement, and wherein subsequently the catalyst compounds in the tubular reactor are removed and replaced by fresh catalyst compounds, and subsequently the tubular reactor is reconnected to the serial arrangement.
